# EUROPEAN PATENT APPLICATION

(11) **EP 1 053 751 A1**
(43) Date of publication of application: **22.11.2000**
(21) Application number: 99401191.4
(22) Date of filing: 17.05.1999
(51) Int. Cl.: A61K 39/395, A61P 35/00

(54) **Compositions and methods for treating cell proliferation disorders**

(71) Applicant: INSTITUT NATIONAL DE LA SANTE ET DE LA RECHERCHE MEDICALE (INSERM), 75643 Paris Cédex 13 (FR); Schering-Plough France, 92307 Levallois-Perret (FR); Institut Curie, 75248 Paris Cedex 05 (FR)
(72) Inventor: Fridman, Wolf Hervé, 75005 Paris (FR); Tartour, Eric, 75017 Paris (FR); Banchereau, Jacques, Dallas, Texas 75226 (US); Fossiez, Francois, 69280 Marcy l'Etoile (FR); Lebecque, Serge J.E., 69380 Civrieux d'Azergues (FR)
(74) Representative: Berger, Helmut

(57) **Abstract**

Pharmaceutical compositions and methods are provided for suppressing tumor growth. The compositions comprise an IL-17 antagonist and/or an IL-17 receptor antagonist and a pharmaceutically acceptable carrier. The compositions are administered in an amount effective to inhibit tumor cell proliferation.

## Description

### Field of the Invention

The invention relates to the field of cytokines, in particular, to the use of cytokine or cytokine receptor antagonists in the treatment of disease states. More specifically, the invention relates to methods of using interleukin-17 (IL-17) and/or IL-17 receptor antagonists to treat cell proliferative diseases such as cancer.

### Background of the Invention

Soluble proteins known as cytokines play a critical role in many aspects of cell differentiation, in cellular interactions, in cellular proliferation and in the development of an immune response.

*In vitro,* interleukin-6 (IL-6) behaves as a growth factor for many tumor cell lines derived from myeloma, lymphoma, Kaposi's sarcoma, melanoma, ovarian and renal or bladder cell carcinoma (Fridman and Tartour 1997, *Molecular aspects of Medicine.* Eds Elsevier Science Ltd, Oxford, England, 18:3-90). In mice, IL-6 transfected tumors often exhibit increased tumorigenicity (Vink *et al.,* 1990, *J. Exp. Med.* **172**:997-1000; Durandy *et al.,* 1994, *J. Immunol.* **152**:5361-5367). In myeloma patients, anti-IL-6 monoclonal antibody administration transiently inhibits myeloma cell proliferation (Bataille *et al.,* 1995, *Blood.* **86**:685-691).

Interleukin-8 (IL-8), a member of the CXC family of chemotactic cytokines, also stimulates the proliferation of tumor cells, as IL-8 is an autocrine growth factor for human melanoma (Schadendorf *et al.,* 1993, *J. Immunol.* **151**:2667-2675). Its expression by tumor cells is directly correlated with their metastatic potential in nude mice (Singh *et al.,* 1994, *Cancer Res.* **54**:3242-3247).

Cervical cancer is associated with human papillomavirus (HPV) infection, but additional factors must contribute to its pathogenesis, since only a minority of HPV infections result in persistent lesions or progress to malignancy (Schiffmank and Brinton, 1995, *Cancer* **76**:1888-1901). Various arguments suggest that IL-6 may be involved in the pathogenesis and development of cervical cancers. IL-6 stimulates the growth of both normal cervical cells, HPV immortalized and cervical carcinoma derived cell lines (Iglesias *et al.,* 1995, *Am. J. Pathol.* **146**:944-952; Eustace *et al.,* 1993, *Gynecol. Oncol.* **50**:15-19.). *In vitro,* cervical carcinoma cells also secrete higher levels of IL-6 and IL-8 than HPV infected and normal cervical epithelial cells (Woodworth and Simpson, *et al.,* 1993, *Am. J. Pathol.* **142**:1544-1555). Increased expression of IL-6 mRNA has been demonstrated in biopsies derived from invasive cervical carcinoma compared to cervical intraepithelial neoplasia or normal cervix (Tartour *et al.,* 1994, *Cancer Res.* **54**:6243-6248).

IL-17, previously termed cytotoxic T lymphocyte associated antigen-8 (CTLA-8), is a 17 kDa cytokine mainly expressed by activated human memory CD4 T cells (Rouvier *et al.,* 1993, *J. Immunol.* **150**:5445-5556; Fossiez *et al.,* 1996, *J. Exp. Med.* **183**:2593-2603; Spriggs, 1997, *J. Clin. Immunol.* **17**:366-369). Its amino acid sequence shares remarkable homology with that of the thirteenth open reading frame (ORF-13) of *Herpesvirus saimiri,* a gamma herpes virus causing T-cell lymphoma in monkey and rabbits (Fleckenstein and Desrosiers, 1992, Herpesviruses, Roizman, ed (New York: Plenum Publishing Press) pp 253-332). *In vitro,* this virus can immortalize human CD4 and CD8 T cells via an unknown mechanism which confers to these cells an IL-2-based autocrine growth following activation by CD2 (Mittrucker *et al.,* 1992, *J. Exp. Med.* **176**:909-913; Biesinger *et al.,* 1992, *Proc. Natl. Acad. Sci. U.S.A.* **89**:3116-3119). IL-17 is considered to be a proinflammatory cytokine since it has been shown to increase the production of interleukin-6 (IL-6) and interleukin-8 (IL-8) in macrophages, fibroblasts, keratinocytes and synovial cells (Fossiez *et al.,* 1996, *J. Exp. Med.* **183**:2593-2603; Yao *et al.,* 1995, *Immunity* **3**:811-821; Chabaud *et al.,* 1998 *J. Immunol.* **161**:409-414) and nitric oxide in human osteoarthritis cartilage via activation of mitogen-activated protein kinase and nuclear factor kappa B (Shalom-Barak *et al.,* 1998, *J. Biol. Chem .* **273**:27467-27473; Attur *et al.,* 1997, *Arthr. Rheum.* **40**:1050-1053). The release of IL-10, IL-12, IL-1R antagonist, and stromelysin is also stimulated by rhIL-17 in human activated macrophages (*Jovanovic et al.,* 1998, *J. Immunol.* **160**:3513-3521).

Characterization of the roles and mechanisms of molecules which induce, sustain, or modulate the various physiological, developmental, or proliferative states of cells will greatly contribute to the development of therapies for conditions which affect the immune system, hematopoietic cells and other cell types. There thus is a need in the art for adequate treatments of diseases characterized by abnormal or inappropriate regulation of the development, physiolgy or proliferation of relevant cell types.

### Summary of the Invention

The present invention fulfills the foregoing need by providing materials and methods for treating disease states characterized by abnormal tumor cell proliferation.

The invention provides a method of treating disease states comprising administering to an individual in need thereof an IL-17 antagonist or an IL-17 receptor antagonist in an amount sufficient to inhibit tumor growth. In preferred embodiments the antagonist is an antibody that binds IL-17 or an anibody that binds IL-17 receptor.

In another aspect of the invention, the IL-17 and/or IL-17 receptor antagonist is administered in combination with other cytokines, and/or with chemotherapuetic agents.

### Brief Description of the Drawing Figures

Figure 1 shows the effect of hIL-17 on the secretion of IL-6 by different tumor cell lines.

Figure 2 shows increased production of IL-6 and IL-8 after *in vitro* treatment of HeLa cells with hIL-17.

Figure 3A shows that IL-17 transfected human tumor cell lines secret IL-17.

Figure 3B shows *in vitro* proliferation of parental and mock or IL-17 transfected human cervical tumor cell lines.

Figures 4A-C show tumor growth in mude mice of mock-transfected or IL-17-transfected human cervical carcinoma cell lines.

### Detailed Description of the Invention

All references cited herein are incorporated in their entirety by reference.

It has now been discovered that IL-17 promotes tumor growth and that the tumor-growth promoting effects of IL-17 can be inhibited by blocking the biological activity of IL-17. The growth-promoting activity of IL-17 can be reduced or eliminated by blocking circulating IL-17 using IL-17 specific antibodies or an antagonist molecule to IL-17. Alternatively, the IL-17 receptor can be blocked using a IL-17 receptor specific antibody or an antagoinist molecule to IL-17 receptor. By blocking IL-17 and/or IL-17 receptor, the growth rate of tumor cells is reduced. Both the naturally occurring and recombinant forms of IL-17 and IL-17 receptor may be used to prepare antibodies for use as antagonist, and for use in drug screening and development.

Both primary and metastatic cancer can be treated in accordance with the invention. Types of cancers which can be treated include but are not limited to melanoma, cervical, breast, pancreatic, colon, lung, glioma, hepatocellular, endometrial, gastric, intestinal, renal, prostate, thyroid, ovarian, testicular, liver, head and neck, colorectal, esophagus, stomach, eye, bladder, glioblastoma, and metastatic carcinomas. The term "carcinoma" refers to malignancies of epithelial or endocrine tissues including respiratory system carcinomas, gastrointestinal system carcinomas, genitourinary system carcinomas, prostatic carcinomas, endocrine system carcinomas, and melanomas. Metastatic, as this term is used herein, is defined as the spread of tumor to a site distant to regional lymph nodes.

Generally, the IL-17 and IL-17 receptor antagonists are administered as pharmaceutical compositions comprising an effective amount of antagonist(s) in a pharmaceutical carrier. These reagents can be combined for therapeutic use with additional active or inert ingredients, e.g., in conventional pharmaceutically acceptable carriers or diluents, e.g., immunogenic adjuvants, along with physiologically innocuous stabilizers and excipients. A pharmaceutical carrier can be any compatible, non-toxic substance suitable for delivering the compositions of the invention to a patient.

The quantities of reagents necessary for effective therapy will depend upon many different factors, including means of administration, target site, physiological state of the patient, and other medicants administered. Thus, treatment dosages should be titrated to optimize safety and efficacy. Animal testing of effective doses for treatment of particular cancers will provide further predictive indication of human dosage. Various considerations are described, e.g., in Gilman *et al.* (eds.) (1990) *Goodman and Gilman's: The Pharmacological Bases of Therapeutics,* 8th Ed., Pergamon Press; and *Remington's Pharmaceutical Sciences,* 17th ed. (1990), Mack Publishing Co., Easton, PA. Methods for administration are discussed therein and below, e.g., for intravenous, intraperitoneal, or intramuscular administration, transdermal diffusion, and others. Pharmaceutically acceptable carriers will include water, saline, buffers, and other compounds described, e.g., in the *Merck Index,* Merck & Co., Rahway, New Jersey. Slow release formulations, or a slow release apparatus may be used for continuous administration.

The preferred biologically active dose of IL-17 and IL-17 receptor antagonists in the practice of the claimed invention is that dosage will induce maximum suppression or inhibition of cell proliferation. The terms suppression and inhibition are used synonomously and refer to the prevention of further growth of an established tumor or the slowing or reduction in the rate of tumor cell proliferation. Generally, treatment is initiated with smaller dosages which are less than the optimum dose of the compound. Thereafter, the dosage is increased by small increments until the optimum effect under the circumstance is reached. Determination of the proper dosage and administration regime for a particular situation is within the skill of the art. Dosage ranges for IL-17 and IL-17 receptor antagonists would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier.

### IL-17 antagonists/IL-17 receptor antagonists

IL-17 antagonists may take the form of proteins which compete for receptor binding, e.g., which lack the ability to activate the receptor while blocking IL-17 binding, or IL-17 binding molecules, e.g., antibodies.

Antibodies can be raised to the IL-17 cytokine, fragments, and analogs, both in their naturally occurring forms and in their recombinant forms. IL-17 polypeptides and nucleic acid sequences encoding them are disclosed in published International Applications WO95/18826 and WO97/15320. Receptors for IL-17 and nucleic acids encoding such receptors are disclosed in U.S. Patent No. 5,869,286. Additionally, antibodies can be raised to IL-17 in either its active forms or in its inactive forms, the difference being that antibodies to the active cytokine are more likely to recognize epitopes which are only present in the active conformation.

Antibodies, including binding fragments and single chain versions, against predetermined fragments of the desired antigens, e.g., cytokine, can be raised by immunization of animals with conjugates of the fragments with immunogenic proteins. Monoclonal antibodies are prepared from cells secreting the desired antibody. These antibodies can be screened for binding to normal or inactive analogs, or screened for agonistic or antagonistic activity. These monoclonal antibodies will usually bind with at least a K_{D} of about 1 mM, more usually at least about 300 µM, typically at least about 10 µM, more typically at least about 30 µM, preferably at least about 10 µM, and more preferably at least about 3 µM or better. The antibodies, including antigen binding fragments, can be potent antagonists that bind to the IL-17 receptor and inhibit ligand binding to the receptor or inhibit the ability of IL-17 to elicit a biological response. IL-17 or fragments may be joined to other materials, particularly polypeptides, as fused or covalently joined polypeptides to be used as immunogens. Although the foregoing addresses IL-17, similar antibodies will be raised against other analogs, IL-17 receptors, and antagonists. Antibodies raised against IL-17 or IL-17 receptors (IL-17R) will also be useful to raise anti-idiotypic antibodies which may be tested for antagonist properties. These will be useful in modulating various cellular responses. The present invention encompasses the use of antibodies that specifically bind IL-17/IL-17R. Antibodies useful in practicing the present invention include polyclonal and monoclonal antibodies. The antibodies may be elicited in an animal host by immunization with IL-17/IL-17R proteins or fragments thereof or may be formed by *in vitro* immunization (sensitization) of immune cells. The immunogenic components used to elicit the production of antibodies may be isolated from cells or chemically synthesized. The antibodies may also be produced in recombinant systems programmed with appropriate antibody-encoding DNA. Alternatively, the antibodies may be constructed by biochemical reconstitution of purified heavy and light chains. The antibodies include hybrid antibodies, chimeric antibodies, humanized antibodies and univalent antibodies. Also included are Fab fragments, including Fab' and F(ab)₂ fragments of antibodies.

Hybridomas of the invention used to make monoclonal antibodies are produced by well-known techniques. Usually, the process involves the fusion of an immortalizing cell line with a B-lymphocyte that produces the desired antibody. Alternatively, non-fusion techniques for generating immortal antibody-producing cell lines are possible, and come within the purview of the present invention, e.g., virally-induced transformation, Casali *et al.,* 1986, Science **234**:476. Immortalizing cell lines are usually transformed mammalian cells, particularly myeloma cells of rodent, bovine, and human origin. Most frequently, rat or mouse myeloma cell lines are employed as a matter of convenience and availability.

Hybridomas are selected by standard procedures, such as HAT (hypoxanthine-aminopterin-thymidine) selection. From among these hybridomas, those secreting the desired antibody are selected by assaying their culture medium by standard immunoassays, such as immunoblotting, ELISA (enzyme-linked immunosorbent assay), RIA (radioimmunoassay), or the like. Antibodies are recovered from the medium using standard protein purification techniques, Tijssen, 1985, *Practice and Theory of Enzyme Immunoassays,* Elsevier, Amsterdam.

### Screening for IL-17/IL-17R antagonists

Both the naturally occurring and recombinant forms of human IL-17/IL-R may be used for drug screening and development. Nucleic acid sequences encoding IL-17 (e.g., cDNA, mRNA) and IL-17 polypeptides can advantageously be used to screen for antagonist, e.g., by screening compounds for IL-17/IL-17R binding activity. IL-17/IL-R or fragments thereof may be used as the basis for low-throughput and high-throughput assays to identify IL-17/IL-R antagonists. The inhibitory agents may comprise nucleic acids, particularly antisense oligonucleotides; peptides; oligosaccharides; lipids; derivatives of any of the foregoing, or other molecules.

The inhibitory agents may be identified using methods well-known in the art, such as, for example, by screening chemical or natural product libraries for the ability to bind to, and/or inhibit or alter the function of the nucleic adds or polypeptides of the invention. Such compounds may be found in, for example, natural product libraries, fermentation libraries (encompassing plants and microorganisms), combinatorial libraries, compound files, and synthetic compound libraries. For example, synthetic compound libraries are commercially available from Maybridge Chemical Co. (Trevillet, Cornwall, UK), Comgenex (Princeton, NJ), Brandon Associates (Merrimack, NH), and Microsource (New Milford, CT). A rare chemical library is available from Aldrich Chemical Company, Inc. (Milwaukee, WI). Alternatively, libraries of natural compounds in the form of bacterial, fungal, plant and animal extracts are available from, for example, Pan Laboratories (Bothell, WA) or MycoSearch (NC), or are readily producible. Additionally, natural and synthetically produced libraries and compounds are readily modified through conventional chemical, physical, and biochemical means (Blondelle *et al.,* 1996, *TibTech* **14**:60). Ligand-binding assays are useful to identify inhibitor compounds that interfere with the function of a particular target protein. These assays are designed to detect binding of test compounds to particular targets. The detection may involve direct measurement of binding. Alternatively, indirect indications of binding may involve stabilization of protein structure or disruption of a biological function. Non-limiting examples of useful ligand-binding assays is the Biomolecular Interaction Assay (BIAcore) system developed by Pharmacia Biosensor and described in the manufacturer's protocol (LKB Pharmacia, Sweden), the yeast two-hybrid system (Fields and Song, 1989, *Nature* **340**:245-246; U.S. Patent No. 5,283,173) and scintillation proximity assays (U.S. Patent No. 4,568,649).

### Pharmaceutical Composititons

Compounds identified as binding to an IL-17/IL-R polypeptide or otherwise interfering with its function are potentially useful as IL-17/IL-R antagonists for use in pharmaceutical compositions.

Once a particular test compound has been identified as a candidate agent, it is tested for two properties: (i) the ability to inhibit tumor growth; and (ii) a lack of effect on different animals. Agents that exhibit tumor growth suppressing activity and a exhibit lack of toxicity for animal cells are especially preferred.

Pharmaceutical formulations suitable for therapy comprise IL-17/IL-R antagonist in conjunction with one or more biologically acceptable carriers. Suitable biologically acceptable carriers include, but are not limited to, phosphate-buffered saline, saline, deionized water, or the like. Preferred biologically acceptable carriers are physiologically or pharmaceutically acceptable carriers.

The compositions include a tumor growth suppressing amount of active agent. This amount will depend upon the agent, the location and nature of the tumor, and the particular host. The amount can be determined by experimentation known in the art, such as by establishing a matrix of dosages and frequencies and comparing a group of experimental units or subjects to each point in the matrix. The therapeutically effective amount can be administered in one administration or over repeated administrations.

The active agents or compositions can be formed into dosage unit forms, such as for example, creams, ointments, lotions, powders, liquids, tablets, capsules, suppositories, sprays, or the like. If the composition is formulated into a dosage unit form, the dosage unit form may contain an effective amount of active agent. Alternatively, the dosage unit form may include less than such an amount if multiple dosage unit forms or multiple dosages are to be used to administer a total dosage of the active agent. Dosage unit forms can include, in addition, one or more excipient(s), diluent(s), disintegrant(s), lubricant(s), plasticizer(s), colorant(s), dosage vehicle(s), absorption enhancer(s), stabilizer(s), bactericide(s), or the like.

For general information concerning formulations, see, *e.g.,* Gilman *et al.* (eds.), 1990, *Goodman and Gilman's: The Pharmacological Basis of Therapeutics,* 8th ed., Pergamon Press; *and Remington's Pharmaceutical Sciences,* 17th ed., 1990, Mack Publishing Co., Easton, PA; Avis *et al.* (eds.), 1993, *Pharmaceutical Dosage Forms: Parenteral Medications,* Dekker, New York; Lieberman *et al.* (eds.), 1990, Pharmaceutical Dosage Forms: Disperse Systems, Dekker, New York.

The compositions can be administered topically or systemically. Topical application is typically achieved by administration of creams, ointments, lotions, or sprays as described above. Systemic administration includes both oral and parental routes. Parenteral routes include, without limitation, subcutaneous, intramuscular, intraperitoneal, intravenous, transdermal, and intranasal administration.

The following examples are intended to further illustrate the present invention without limiting its scope.

### EXAMPLES

Various materials and methods used in Examples 1-5 are described below.

### Tumor Cell Lines

The HeLa cervical tumor cell line was obtained from the American Type Culture Collection (Manassas, VA). The IC1 cervical carcinoma cell line was provided by Dr J. Couturier (Couturier *et al.,* 1991, *J. Virol.* **65**:4534-4538). The human melanoma cell lines WM 793, 1341D, MZ2 and HT144 have been described by Montero *et al.,* 1997, *Clin. Cancer Res.* **3**:1443-1451. All cell lines were grown in RPMI 1640 medium (Biowhittaker, Walkersville, MD) supplemented with 10% Fetal Calf Serum (FCS), and 100 U/ml penicillin-streptomycin, 5% sodium pyruvate, and 0.01% mercaptoethanol (all from Sigma Chemical Co., St Louis, MO).

### Transfection of HeLa and IC1 Cells

A cDNA encoding hIL-17 was inserted into an expression vector under the control of the SRα promoter in pBR322 into which a neomycin resistance gene was introduced (NT-Neo) (Daeron *et al.,* 1995, *Immunity* **3**:635-646). NT-Neo, containing or not containing the 640-bp IL-17 cDNA, were linearized with *Sca* I restriction enzyme, and stably transfected by electroporation into HeLa and IC1 cells. Electroporation was performed with a Bio-Rad Gene Pulser, at a voltage of 260 V and with a capacitance of 960 mF. Seventy-two hours after electroporation, transfectants were selected by culture in RPMI medium (Gibco) supplemented with 1 mg/ml of G418 (Geneticin) from Gibco BRL (Paisley, Scotland). G418-resistant clones were expanded in selection medium and tested for IL-17 expression.

### Recombinant Proteins and Antibodies

Recombinant hIL-17 was purified from the supernatant of IL-17 transfected NSO cells as described by Fossiez *et al.,* 1996, *J. Exp. Med.* **183**:2593-2603. Neutralizing anti-IL-17 mAb 5 as well as anti-IL17 mAb 16 and 25 were produced in ascites and purified by anion-exchange chromatography (Fossiez *et al.,* 1996, *J. Exp. Med.* **183**:2593-2603).

### Assays

Human IL-6 and IL-8 were assayed using enzyme-linked immunosorbent assay (ELISA) kits purchased from Immunotech (Marseille, France) and Medgenix (Brussels, Belgium), respectively.

An ELISA was used to measure human IL-17 concentrations in culture supernatants. Briefly, ninety-six break-away, flat-bottomed-well (Nunc) microtiter plates were coated with 50 ml of anti-IL-17 mAb 25 (10 mg/ml) diluted in carbonate buffer (0.1 M Na₂CO₃/NaHCO₃, pH 9.6) overnight at 4°C.

The plate was then saturated with 200 ml of PBS-1% BSA for 1h at room temperature. After washes with PBS-0.05% Tween 20 (Merck, Schuchardt, Germany), 50 ml of recombinant hIL-17 or samples diluted in PBS-1% BSA were added and incubated for 3 hours at 37°C. After washes, the plates were then incubated with 50 ml of peroxidase-labeled anti-IL-17 mAb 16 (5mg/ml) diluted in PBS-1% BSA for 2h at 37°C. The reaction was revealed by addition of the peroxidase substrate (o-phenylenediamine-dihydrochloride) and the OD was read at a wavelength of 492 nm. The lowest concentration of human IL-17 detected was 0.05 ng/ml.

### Reverse Transcriptase-PCR Amplification

RT-PCR was performed as described by Tartour *et al.,* 1998, *J. Natl. Cancer Inst.* **4**:287-294. The following oligonucleotide primers were used: Human (h) b actin sense (TCGTCGACAACGGCTCCGG CATGTGC), hb actin antisense (TTCTGCAGGGAGGAGCTGGAAGCAGC), hIL-6 sense (ACGAATT CACAAACAAATTCGGTACA), hIL-6 antisense (CATCTAGATTCTTTGCCT TTTTCTGC), hIL-8 sense (TTCTGCAGCTCTGTGTGAAGG), hIL-8 antisense (GAAGAGGGCTGAGAATTCAT), hIL-17 sense (ACTCCTGGGAAGACCTC ATTG), hIL-17 antisense (GGCCACATGGTGGACAATCG), hCD4 sense (GGAGTCCCTTTTAGGCACTTGC), hCD4 antisense (GAACTCCACCTGTTC CCCCTC), hCD8 sense (CTCCTCCTGGCCGCGCAGCTG), hCD8 antisense (GCCGGGCTCTCCTCCGCCG), murine (m) IL-6 sense (TGGAGTCACAGA AGGAGTGGCTAAG), mIL-6 antisense (TCTGACCACAGTGAGGAAT GTCCAC), m-Hypoxanthine-guanine phosphoribosyl transferase (HPRT) sense (GTTGGATACAGGCCAGACTTTGTTG), mHPRT antisense (GATTC AACTTGCGCTCATCTTAGGC). (SEQ ID NOS. 1-16, respectively).

### In vitro Growth Assay

Cells were plated into flat-bottomed ninety-six well plates at a density of 10⁴ cells per well. The cells were cultured for 3 days and their proliferation was determined by a MTT assay (Hansen *et al.,* 1989, J. *Immunol. Meth.* **119**:203-210). Briefly, 20 ml of MTT (4,5-dimethylthiazol-2-yl) -2,5 diphenyl tetrazolium bromide (Sigma) at a concentration of 5 mg/ml were added to each well and incubated for 4 hours in the dark. The formazan grain was then dissolved in dimethyl sulfoxide and the absorbance at 570 nm was read using an ELISA plate reader. A standard curve between the absorbance of the MTT test and the number of cells was determined for each cell line. The conversion of MTT to formazan was directly correlated with the number of viable cells.

### Tumor Growth in Nude Mice

Eight-week-old male athymic nu/nu mice (Iffa Credo, L'Arbresle, France) were used in the described experiments. Human cervical tumors were injected into mice by subdermal inoculation of 10⁶ cells. Eight to ten mice per group were used per experiment. Tumor volume (in mm3) was estimated from the length (a) and width (b) of the tumor by the formula: volume = ab²/2. Biopsies were snap-frozen in liquid nitrogen and stored at -70°C for RNA extraction.

### Immunocytochemistry

Five mM cryostat sections of tumor xenografts were fixed in acetone at 4°C for 5 min. After washes in Tris Buffer Saline (TBS), they were incubated with biotinylated rat mAb against Mac 1 (M1/70 hybridoma) or isotype-matched control biotinylated rat mAb (Pharmingen, San Diego, SA). After washes in TBS, slides were subsequently incubated with alkaline phosphatase-conjugated streptavidin (Dako, Trappes, France) and enzymatic activity was revealed with Fast Red reagent (Dako) associated with 1 mM of Levamisole, a known inhibitor of endogenous alkaline phosphatase. Sections were counterstained with Mayer's Hematoxylin.

### Statistical Analysis

The *in vivo* comparison of tumor size between the various groups of mice was analyzed by the Mann-Whitney test.

### Example 1

### IL-6 and IL-8 production by tumor cell lines stimulated with rhIL-17

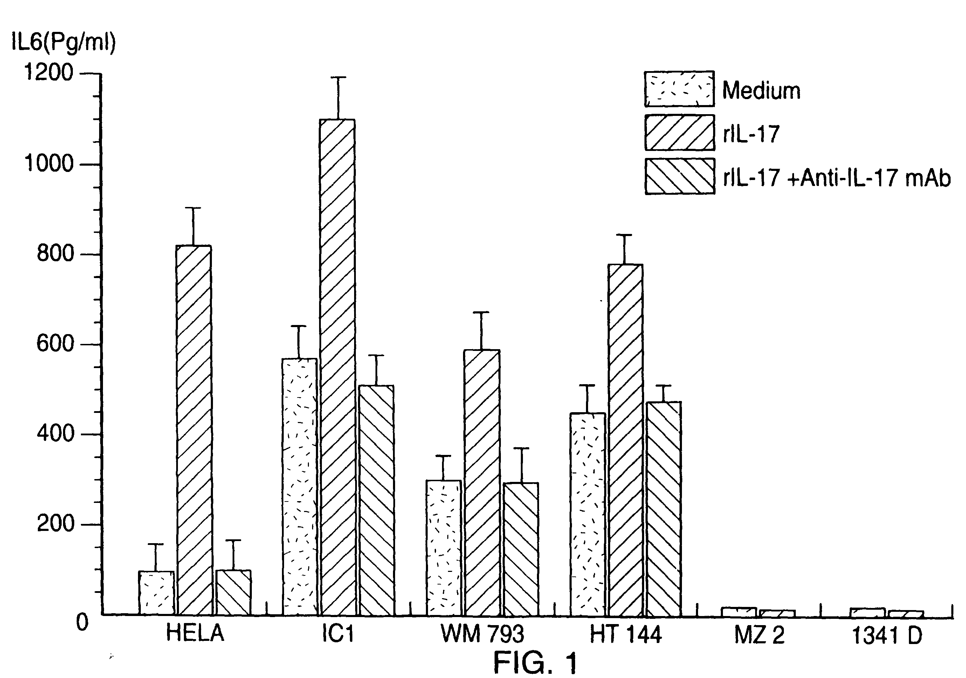

10⁶ human cervical carcinoma cells (HeLa or IC1) or melanoma cells (WM793, HT144, MZ2, 1341D) were added per well in a 24-well plate and stimulated with recombinant hIL-17 (100 ng/ml), a mix of hIL-17 (100 ng/ml) preincubated during 30 min at 37°C with 1 mg/ml of neutralizing anti hIL-17 mAb5, or culture medium alone. After 24 hours, culture supernatants were harvested and tested for IL-6 concentrations using a commercially available ELISA. Figure 1 shows the effect of hIL-17 on the secretion of IL-6 by different tumor cell lines. The results represent the mean ± SD of triplicate determinations from one representative experiment.

As can be seen in Figure 1, recombinant human IL-17 (rhIL-17) significantly increased IL-6 secretion by the two human cervical cell lines, Hela and IC1. This action was not restricted to tumor cell lines derived from cervical carcinoma, since two out of the four melanoma cell lines tested were also sensitive to rIL-17. Addition of anti-IL-17 mAb inhibited up-regulation of IL-6 production by IL-17. Tumor cells did not constitutively secrete significant levels of IL-17 and anti-IL17 antibody alone had no effect on IL-6 secretion. Only cell lines which already produced basal levels of IL-6 appeared to respond to IL-17.

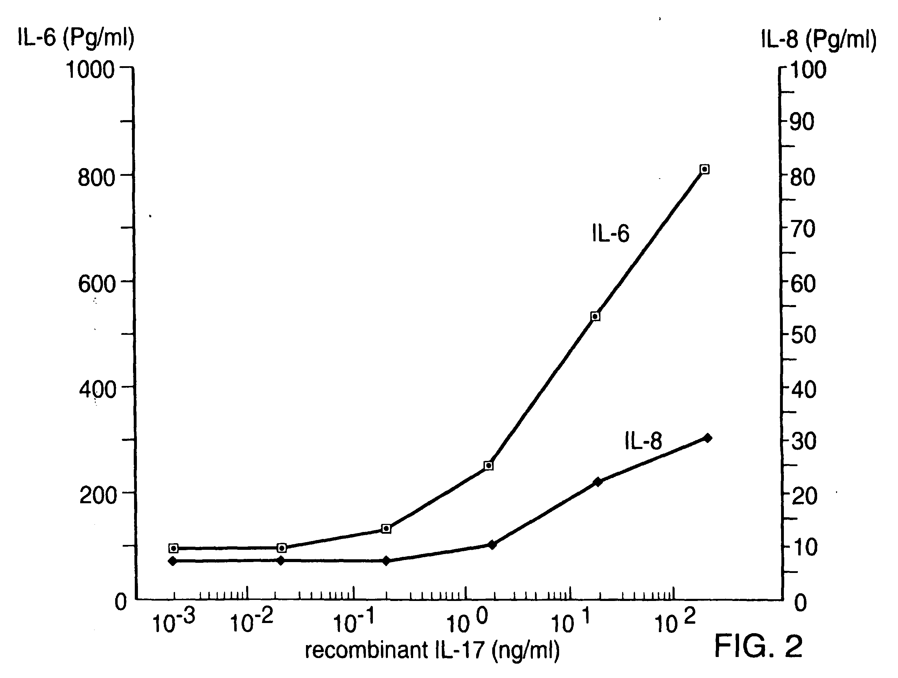

An increased production of IL-8 was also demonstrated after treatment of HeLa, IC1, WM793 and HT144 cell lines with rIL-17. Figure 2 shows an increased production of IL-6 and IL-8 after *in vitro* treatment of HeLa cells with hIL-17. In the experiment reported in Figure 2, the human cervical carcinoma cell line, HeLa, was plated at 10⁶ cells per ml and cultured with various concentrations of purified hIL-17. After 24 hours the levels of IL-6 ( 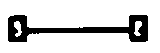 ) and IL-8 ( 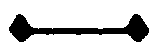 ) in the supernatants were measured by ELISA. In contrast, cell lines resistant to rIL-17-induced IL-6 production (MZ2, WM35) did not secrete IL-8 after IL-17 treatment. The activity of rIL-17 was observed at a dose range between 0.2 and 2 ng/ml. No effect of IL-17 on G-CSF production, which has been shown to be regulated by IL-17 in other cell types (Fossiez *et al.,* 1996, *J. Exp. Med.* **183**:2593-2603; Cai *et al.,* 1998, *Immunol. Letters* **62**:51-58), was observed.

IL-6 and IL-8 mRNA expression was assessed by RT-PCR in rIL-17-treated and untreated cervical carcinoma cells in order to more thoroughly analyze the mechanisms of action of IL-17. cDNA derived from mRNA extracted from HeLa cells was cultured for 6 hours with medium alone or hIL-17 (10 and 100 ng/ml), was amplified by PCR using primers specific for β actin, IL-6 and IL-8 mRNA. Amplified PCR products were then loaded onto a 2% agarose gel and stained with ethidium bromide for ultraviolet visualization. A clear upregulation of IL-6 and IL-8 mRNA was observed 6 hours after *in vitro* stimulation of HeLa cells with rIL-17. IL-17 therefore appears to regulate IL-6 and IL-8 either at the transcriptional level or by increasing their mRNA half life.

### Example 2

### Characterization of IL-17 transfected human cervical carcinoma cell lines

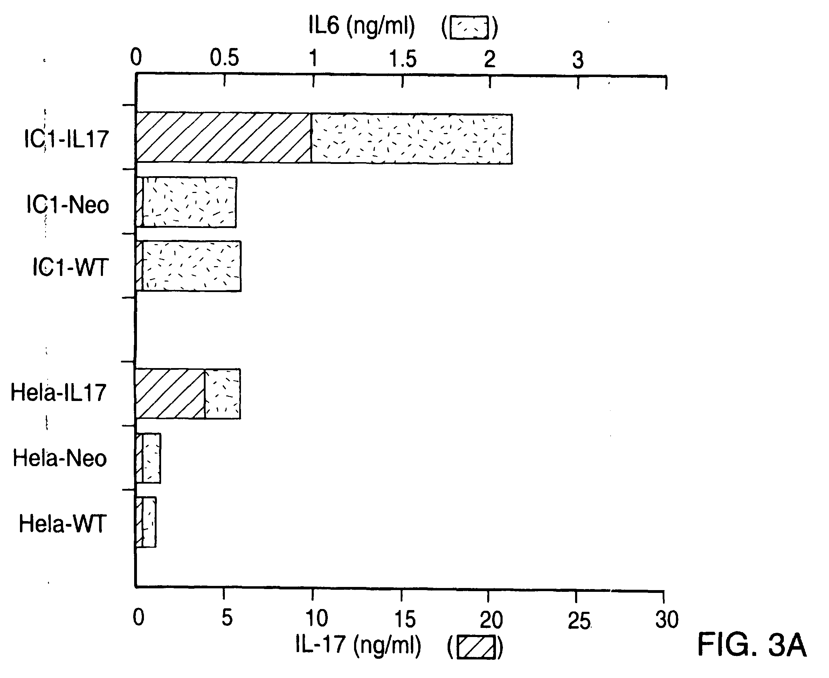

To further analyze the role of IL-17 on human cervical carcinoma cell lines, two tumor cell lines (Hela, IC1) were transfected with a cDNA encoding human IL-17. Wild type (WT) or human cervical carcinoma cells (HeLa , IC1) transfected with NT plasmid alone (Neo) or cDNA encoding human IL-17 were plated in 24-well flat-bottom plates at a density of 10⁶ cells per well. The cells were cultured for 24 hours and the IL-6 ( 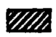 ) or IL-17 ( 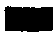 ) levels were determined by ELISA. As shown in Figure 3A, stable transfectants were obtained which secreted significant amounts of IL-17 in their supernatant, from 4 ng/ml to 10 ng/ml for 5 X 10⁵ cells over 24 hours. No expression of either IL-17 mRNA or protein could be detected in any of the tumor cell lines analyzed prior to transfection. Interestingly, IL-17 transfected-cell lines produced more IL-6 than the parent cervical carcinoma cell lines or cells transfected with the vector alone, which confirms results obtained with rIL-17. No clear increase of IL8 secretion was observed in these IL-17-transfectants, which could explain by the weaker effect of IL-17 in the regulation of IL-8 than IL-6 in these cells, as shown in Figure 2.

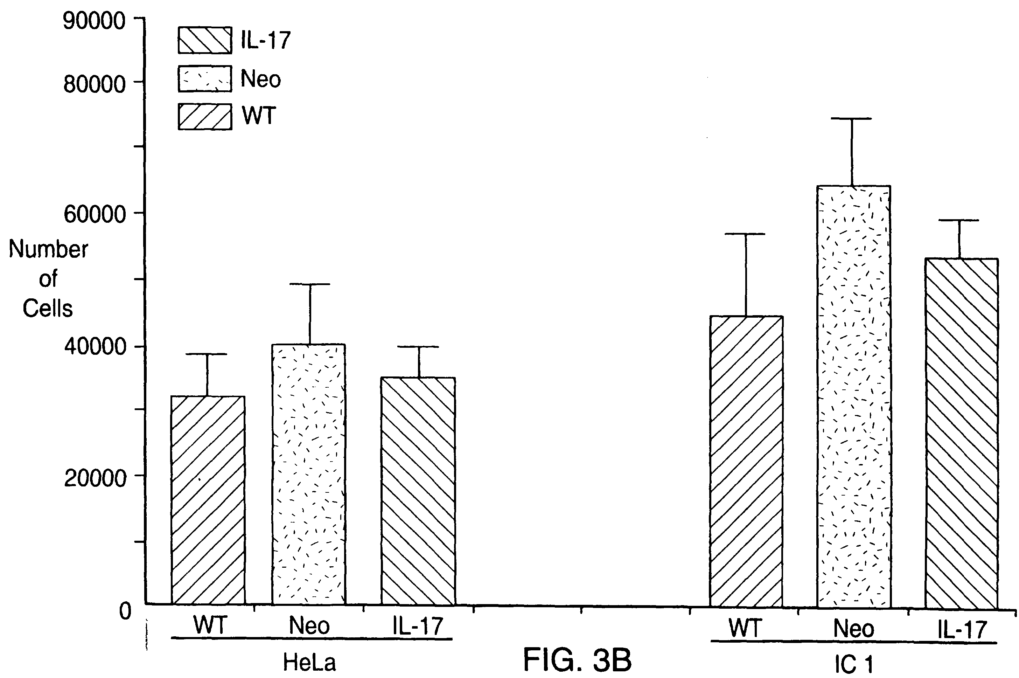

The growth rate of the various clones was also investigated. Wild type (WT) or human cervical carcinoma cells (HeLa, IC1) transfected with NT plasmid alone (Neo) or cDNA encoding human IL-17 were plated in 96-well flat-bottom plates at a density of 10⁴ cells per well. The cells were cultured for 3 days and their proliferation was determined by a MTT assay. For these experiments, all cells were cultured in the same medium without G418/Neomycin. For each cell line, a standard curve between the absorbance of the MTT test and the number of cells was determined. Figure 3B shows a typical experiment, in which IL-17-expressing and parent cells (Hela, IC1) showed identical in *vitro* growth. Values are mean ± SD (bars) of triplicate culture and are representative of 2 experiments. Similarly, exogenous recombinant IL-17 did not influence the proliferation of human cervical cell lines.

### Example 3

### Tumor growth of human cervical carcinoma cells and their IL-17 transfectants in nude mice

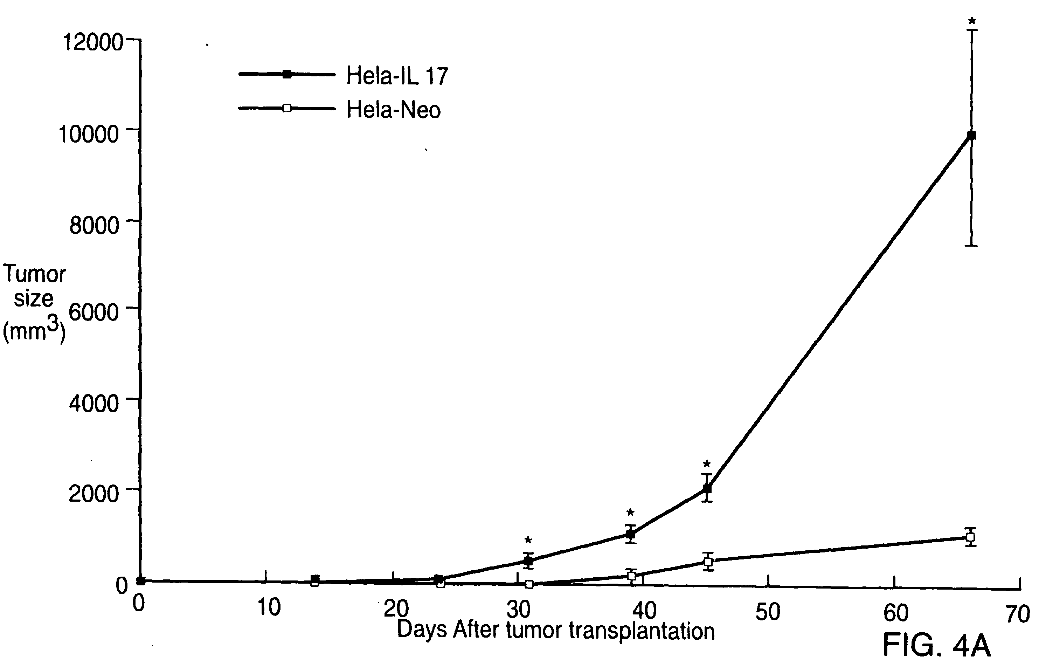

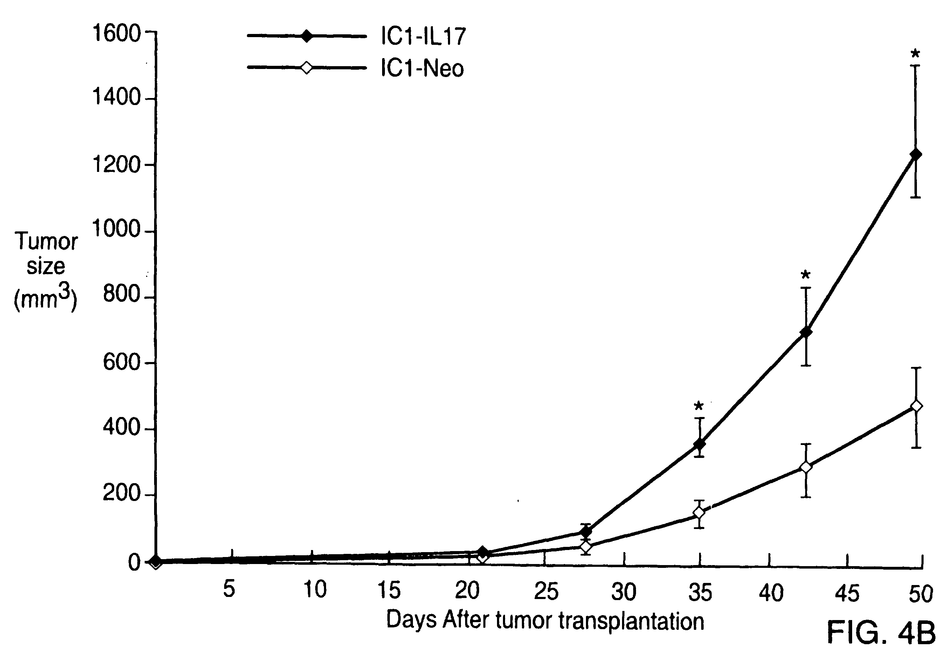

In contrast to the absence of any significant effect of IL-17 on *in vitro* growth of cervical carcinoma cell lines, the two cervical carcinoma cell lines (HeLa and IC1), transfected with human IL-17 cDNA and then transplanted in nude mice, were found to grow faster than mock-transfected cells (Figures 4A and 4B).

The mock- (Neo) or IL-17-transfected human cervical cell line HeLa (Figure 4A) or IC1 (Figure 4B) was injected into mice by subdermal inoculation of 10⁶ cells. Eight to ten mice per group were used per experiment and tumor size was assessed as described in the Material and Methods detailed above. Results are representative of two independent experiments. For example, at day 31 after transplantation, a mean tumor volume of 26 mm³ was measured in transplanted parental Hela cells, while the tumor volume of the IL-17-transfected Hela cell line reached 404 mm³ (p = 0.0003) (statistically significant results p < 0.05).

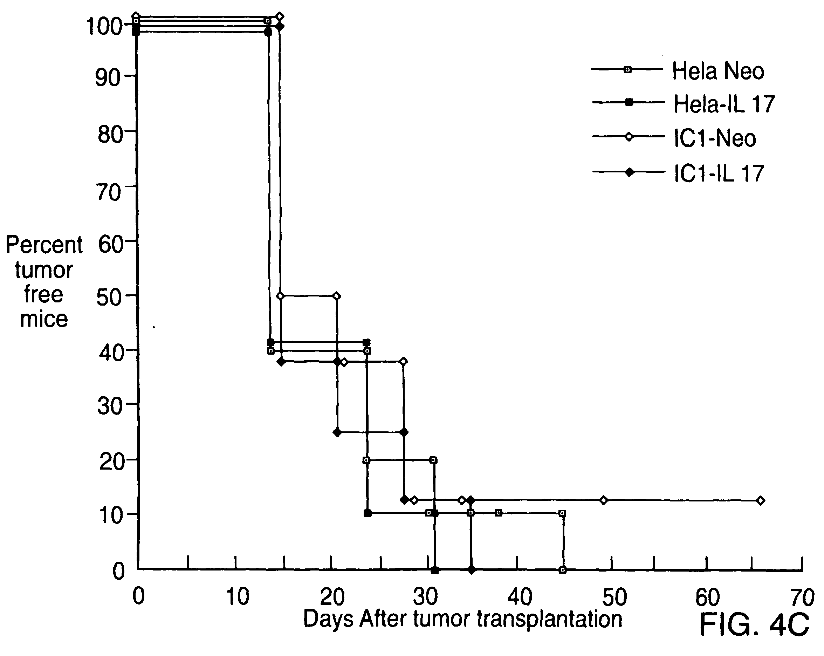

IL-17 did not affect the tumor incidence rate (Figure 4 C) and its effect seemed more pronounced after an initial growth period in nude mice. IL-17 therefore did not play a role in the initial stage of tumor development, but rather enhanced its progression.

### Example 4

### Analysis of the expression and activity of the human IL-17 transgene in nude mice

To determine the *in vivo* persistence of the IL-17 transgene in tumors transfected with IL-17 cDNA and transplanted in nude mice, biopsies were performed at day 56 after transplantation. cDNA derived from mRNA extracted from mock- (Hela-Neo) or IL-17-transfected (Hela-IL-17) cells were amplified by PCR using primers specific for human IL-17 murine IL-6 and murine HGPRT mRNA. Amplified PCR products were loaded onto a 2% agarose gel and stained with ethidium bromide for ultraviolet visualization. Human IL-17 cDNA was detected in 4/4 biopsies derived from mice transplanted with HeLa-IL-17 cells, but not in mice previously injected with the control transfectant (HeLa-Neo), indicating that the transgene was not lost during tumor development.

Since various studies have reported that human IL-17 is biologically active in murine cells (Kennedy *et al.,* 1996, *J. Interf. Cyt. Res.* **16**:611-617), the *in situ* expression of genes normally regulated by IL-17, such as IL-6, were analyzed. Increased expression of murine IL-6 mRNA was demonstrated in biopsies excised from HeLa-IL-17 cells. Murine IL-6 primers used for this study did not cross-react with human interleukin-6. The same analysis could not be performed for human IL-6 mRNA because the primers used could also amplify murine IL-6 mRNA. The murine counterpart of human IL-8 has not yet been isolated and its expression was therefore not investigated.

Cryostat section of biopsies derived from IL-17- or mock- transfected HeLa cervical carcinoma transplanted in nude mice were fixed in acetone and incubated with biotinylated rat anti Mac 1 or isotype matched control biotinylated rat mAb. After washes in TBS, slides were subsequently incubated with alkaline phosphatase-conjugated streptavidin and enzymatic activity revealed with Fast Red reagent. Sections were counterstained with Mayer's Hematoxilin. Immunohistochemical analysis revealed marked infiltration by murine macrophages of HeLa-IL-17 tumors, whereas mock-transfected HeLa tumors did not seem to recruit cells from the monocyte-macrophage lineage. This selective peri and intratumoral macrophage recruitment was also detected in IC1-IL17 tumors using other antibodies (anti-Mac3) recognizing antigenic determinants on macrophages. The intratumoral infiltration of other immune cells such as T cells, B cells and neutrophils did not seem to differ in tumor xenografts derived from mock or IL-17 transfected HeLa cells.

### Example 5

### Human IL-17 mRNA expression in biopsies derived from invasive cervical carcinomas

A study was conducted to determine whether IL-17 is expressed in tumor biopsies from cervical carcinoma patients. Six cDNA derived from mRNA extracted from 6 primary invasive cervical carcinomas were amplified by PCR using oligonucleotide primers specific for human IL-17, CD4 and CD8. Amplified PCR products were loaded onto a 2% agarose gel and stained with ethidium bromide for ultraviolet visualization. Significant expression of IL-17 was shown in 4/6 samples tested. No IL-17 mRNA expression could be observed in tumors only infiltrated by CD8 T cells. Equivalent β actin mRNA in the various samples assessed by RT-PCR excluded a bias in the interpretation of these results due to variation of total mRNA levels. The presence of CD4 positive T cells seemed to be associated with IL-17 mRNA expression, in line with previous studies indicating the activated memory CD4 origin of this cytokine (Fossiez *et al.,* 1996, *J. Exp. Med.* **183**:2593-2603).

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

## Claims

1. A method of suppressing tumor cell proliferation in a mammal comprising administering an IL-17 antagonist and/or IL-17 receptor antagonist in an amount effective to suppress proliferation of tumor cells.

2. The method of claim 1 wherein the mammal is human.

3. The method of claim 1 wherein the IL-17 antagonist is a monoclonal antibody that binds IL-17.

4. The method of claim 1 wherein the IL-17 receptor antagonist is a monoclonal antibody that binds an IL-17 receptor.

5. A parmaceutical compositions for suppressing tumor growth comprising an IL-17 antagonist and a pharmaceutically acceptable carrier.

6. The composition of claim 5 wherein the IL-17 antagonist is a monoclonal antibody that binds IL-17.

7. A parmaceutical compositions for suppressing tumor growth comprising an IL-17 receptor antagonist and a pharmaceutically acceptable carrier.

8. The composition of claim 7 wherein the IL-17 receptor antagonist is a monoclonal antibody that binds an IL-17 receptor.


### Annex to the application documents - subsequently filed sequences listing

SEQUENCE LISTING

<110> INSERM
      INSTITUT CURIE
      SCHERING-PLOUGH FRANCE

<120> Compositions and Methods for Treating Cell
      Proliferation Disorders

<130> SF0941 EP

<140> EP 99 401 191.4
<141> 1999-05-17

<160> 16

<170> PatentIn Ver. 2.1

<210> 1
<211> 26
<212> DNA
<213> Homo sapiens

<400> 1
tcgtcgacaa cggctccggc atgtgc                                      26


<210> 2
<211> 26
<212> DNA
<213> Homo sapiens

<400> 2
ttctgcaggg aggagctgga agcagc                                      26


<210> 3
<211> 26
<212> DNA
<213> Homo sapiens

<400> 3
acgaattcac aaacaaattc ggtaca                                      26


<210> 4
<211> 26
<212> DNA
<213> Homo sapiens

<400> 4
catctagatt ctttgccttt ttctgc                                      26


<210> 5
<211> 21
<212> DNA
<213> Homo sapiens

<400> 5
ttctgcagct ctgtgtgaag g                                           21

<210> 6
<211> 20
<212> DNA
<213> Homo sapiens

<400> 6
gaagagggct gagaattcat                                                    20


<210> 7
<211> 21
<212> DNA
<213> Homo sapiens

<400> 7
actcctggga agacctcatt g                                                  21


<210> 8
<211> 20
<212> DNA
<213> Homo sapiens

<400> 8
ggccacatgg tggacaatcg                                                    20


<210> 9
<211> 22
<212> DNA
<213> Homo sapiens

<400> 9
ggagtccctt ttaggcactt gc                                                 22


<210> 10
<211> 21
<212> DNA
<213> Homo sapiens

<400> 10
gaactccacc tgttccccct c                                                  21


<210> 11
<211> 21
<212> DNA
<213> Homo sapiens

<400> 11
ctcctcctgg ccgcgcagct g                                                  21


<210> 12
<211> 19
<212> DNA
<213> Homo sapiens

<400> 12
gccgggctct cctccgccg                                                     19

<210> 13
<211> 25
<212> DNA
<213> Murine

<400> 13
tggagtcaca gaaggagtgg ctaag                                                25

<210> 14
<211> 25
<212> DNA
<213> Murine

<400> 14
tctgaccaca gtgaggaatg tccac                                                25

<210> 15
<211> 25
<212> DNA
<213> Murine

<400> 15
gttggataca ggccagactt tgttg                                                25

<210> 16
<211> 25
<212> DNA
<213> Murine

<400> 16
gattcaactt gcgctcatct taggc                                                25